# FASCICULE DE BREVET EUROPEEN

(11) **EP 2 422 954 B1**
(45) Date de publication et mention de la délivrance du brevet: **10.04.2013**
(21) Numéro de dépôt: 11189032.3
(22) Date de dépôt: 18.11.2008
(51) Int. Cl.: B29C 49/42, B29C 49/78, B29C 49/06

(54) **Dispositif de transport d'un corps creux, installation pourvue de tels dispositifs et procédé de convoyage d'un corps creux solidarisé à un tel dispositif.**
Transportvorrichtung für einen Hohlkörper, mit solchen Vorrichtungen ausgestattete Anlage und Beförderungsverfahren eines mit einer solchen Vorrichtung fest verbundenen Hohlkörpers
Device for transporting a hollow body, facility provided with such devices and method for transporting a hollow body secured to such a device.

(30) Priorité: 19.11.2007 FR 0708110
(43) Date de publication de la demande: 29.02.2012
(62) Demande divisionnaire de: 08857219.3
(73) Titulaire: Sidel Participations, 76930 Octeville sur Mer (FR)
(72) Inventeur: Mie Patrick, 76930 Octeville s/mer (FR); LETHUILLIER Frédéric, 76930 OCTEVILLE-SUR-MER (FR)
(74) Mandataire: Cabinet Plasseraud

(56) Documents cités:
- FR-A1- 2 766 121
- FR-A1- 2 774 912
- FR-A1- 2 887 525
- JP-A- 11 048 323
- US-A- 4 890 726

## Description

L'invention se rapporte de manière générale au domaine de fabrication de récipients en matériau thermoplastique obtenus après chauffage et soufflage d'un corps creux appelé communément préforme, et elle porte plus particulièrement sur un procédé de convoyage d'un corps solidarisé à un dispositif de transport, et sur un dispositif de transport d'un corps creux.

Ainsi, l'invention s'applique aux installations de traitement en ligne pour la fabrication ou le traitement de préformes en matière thermoplastique telle que du PET, dans lesquelles installations lesdites préformes sont déplacées individuellement les unes à la suite des autres à l'aide de dispositifs de transport aptes à les maintenir par leur col, de manière à laisser leur corps dégagé en vue du chauffage.

De manière courante, une préforme se présente sous la forme générale d'un corps cylindrique tubulaire fermé à l'une de ses extrémités axiales et prolongé à son autre extrémité ouverte par un col présentant déjà la forme définitive du col du récipient final ainsi qu'une collerette s'étendant sensiblement radialement. Dans la suite de la description, il est entendu par « récipient pourvu d'un col », tout corps creux présentant un col, à savoir des préformes, à partir desquelles sont fabriqués des récipients par soufflage ou étirage-soufflage, c'est-à-dire des récipients ayant leur forme définitive et devant subir un traitement additionnel (tel qu'une étape d'étiquetage ou de remplissage).

Dans le cas particulier du transport de préformes, en vue de leur mise en température par chauffage préalablement à l'étape de soufflage, il est nécessaire de prévoir un dispositif de transfert permettant de prendre chaque préforme par son col (« vêtissage ») pour la déplacer dans une zone de chauffage, en général dans un four tunnel de conditionnement thermique, dans laquelle zone le corps de ladite préforme doit être chauffé à la température de transition vitreuse de la matière thermoplastique, tout en restant à une température inférieure à la température de cristallisation.

Pour réaliser ce transfert, il est connu d'utiliser des dispositifs de transport munis d'organes de préhension qui comportent un mandrin propre à être engagé à l'intérieur du col de la préforme (vêtissage intérieur) et qui maintient cette dernière simplement par adhérence du fait de la présence des forces de pression et de frottement (voir par exemple US 4 890 726 et JP 11 048 323).

Il est présenté, dans le document FR 2 706 876, au nom de la Demanderesse, un dispositif de vêtissage intérieur de préformes à partir d'une tête de préhension ayant une extrémité à bague fendue.

Selon le même principe du vêtissage intérieur, il est connu, comme présenté dans le document FR 2 794 109 au nom de la Demanderesse, de réaliser un système de convoyage de préformes formé d'un ensemble d'éléments de transport comprenant chacun un dispositif de préhension muni à son extrémité d'une bague annulaire fendue formée de plusieurs secteurs ; lorsque la bague est engagée intérieurement dans le col d'une préforme, les secteurs qui sont sollicités radialement vers l'extérieur, par des moyens élastiques, sont aptes à prendre appui sur la surface intérieure cylindrique du col.

De manière alternative, il est également possible que la préforme soit saisie au niveau de la face externe de son col (vêtissage extérieur), comme décrit, par exemple, dans le document FR 2 882 963 au nom de la Demanderesse.

Dans les installations de traitement en ligne pour la fabrication récipients ou le traitement de corps creux, il est parfois nécessaire de faire subir au volume intérieur des corps creux des actions de traitement à l'aide de fluide et/ou des interactions appropriées avec des fluides, par exemple des actions de stérilisation ou d'aseptisation par injection de fluide aseptique (voir par exemple FR 2 774 912).

Ces interactions sont généralisées en amont ou en aval du dispositif de transport, dans la mesure où ses organes de transport viennent obturer le corps creux.

Lors du passage de cette préforme dans le four de conditionnement thermique, la préforme subit une rotation sur elle-même afin de permettre un chauffage homogène sur toute la surface extérieure de la préforme. Or, lors du vêtissage d'une préforme, aussi bien intérieur qu'extérieur, il est possible que celle-ci ne soit pas axialement alignée avec la tête de préhension.

Dans ce cas, du fait du désaxage de la préforme et de son mouvement de rotation sur elle-même, le corps de la préforme, et en particulier son extrémité, est susceptible de venir en contact avec les lampes de chauffage du four et de causer des dégâts importants en les cassant, et aussi, parfois, la préforme peut s'enflammer.

Ainsi, afin de prévenir ces incidents, comme la détérioration des lampes de chauffage du four, il est nécessaire de prévoir, avant leur entrée dans le four, une opération de dévêtissage des préformes, c'est-à-dire une opération qui consiste à éjecter toutes les préformes qui sont susceptibles de générer des incidents lors de leur passage dans le four de chauffage.

Cette opération de dévêtissage permet, d'une manière générale, d'éviter de chauffer des préformes qui présenteraient un défaut de conception en entrée du four.

Le document FR 2 872 805 au nom de la Demanderesse, décrit un système de dévêtissage de préformes.

Des moyens de détection optique sont prévus pour déterminer quelles sont les préformes à dévêtir et, sous l'effet d'un appareil de commande approprié, du genre automate, un organe d'éjection intervient pour dégager les préformes en question, qui tombent alors dans un bac de récupération, par exemple.

Ce système, bien que donnant entière satisfaction, est néanmoins complexe à mettre en oeuvre et, surtout, il bride les cadences de production de l'installation ; en effet, le temps de réponse de l'organe d'éjection, en vue du dévêtissage, est relativement long.

De plus, du fait des cadences de production actuelle et donc de la vitesse de défilement des préformes, il n'est pas possible d'éjecter une seule préforme car, du fait du temps de réponse des moyens pneumatiques de commande, ce sont plusieurs préformes qui sont touchées par l'organe d'éjection et, en général, de manière courante, ce sont trois préformes successives qui, en moyenne, sont éjectées.

Du fait du coût de fabrication des préformes, et afin d'empêcher la mise au rebut de préformes correctement placées sur la tête de vêtissage ou ne présentant aucun défaut, il serait particulièrement intéressant de réaliser un dispositif de dévêtissage de préformes permettant le dévêtissage uniquement de la préforme mal solidarisée ou mal formée ; ledit dispositif pouvant être mis en oeuvre à des cadences élevées de circulation des préformes. Il serait également particulièrement intéressant de réaliser un système de dévêtissage pouvant également s'appliquer au dévêtissage des préformes mais et aussi à tout type de corps creux.

De plus, du fait de ce dévêtissage de plusieurs préformes disposées les unes à côté des autres, il existe des espaces libres importants dans la chaîne de convoyage des préformes (trois têtes de vêtissage successives). Ces grands espaces entre les préformes, dans le four, perturbent la chauffe des autres préformes lors de leur passage devant les lampes dudit four ; il en résulte des défauts d'homogénéité du chauffage des préformes.

Par ailleurs, une évolution actuelle dans le cadre de la fabrication des préformes, tend à diminuer les dimensions de la collerette, laquelle devient de moins en moins épaisse et de moins en moins large, d'où des problèmes de vêtissage plus fréquents.

Ainsi, il serait également particulièrement avantageux de réaliser un système de dévêtissage qui ne soit pas dépendant de la taille d'une collerette prévue sur le col du corps creux.

La présente invention propose donc de résoudre les différents problèmes liés à l'art antérieur à l'aide d'un système de dévêtissage de corps creux permettant de dévêtir une seule préforme à des cadences de convoyage élevées, lequel système, en plus, ne soit pas des caractéristiques dimensionnelles de la collerette de ladite préforme.

Selon un premier de ses aspects, la présente invention concerne un procédé de convoyage d'un corps creux solidarisé à un dispositif de transport selon la revendication 1.

L'invention concerne également un dispositif de transport d'un corps creux selon la revendication 3.

Les revendications dépendantes montrent des modes particuliers de réalisation.

La présente invention est maintenant décrite à l'aide d'un exemple uniquement illustratif et nullement limitatif de la portée de la présente invention, et à partir de la figure suivante qui représente une vue en coupe axiale d'un dispositif de transport et de vêtissage d'un corps creux selon l'invention, lequel dispositif, en coopération avec une buse de soufflage, permet d'effectuer une opération de dévêtissage des préformes.

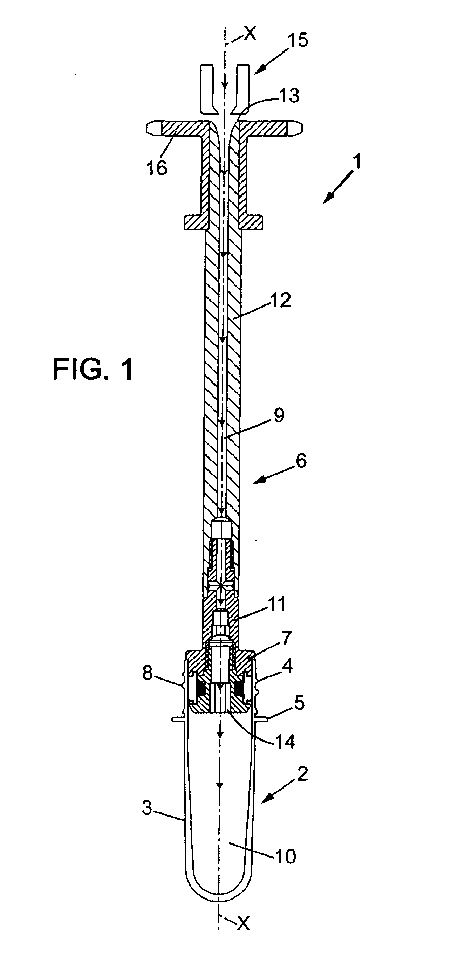

De manière simplifiée, la figure 1 représente une vue en coupe axiale d'un dispositif 1 de transport d'un corps 2 en matière thermoplastique. La préforme 2 présente un corps 3 s'étendant de manière sensiblement longitudinale, et un col 4 séparé du corps 3 par une collerette 5 s'étendant selon une direction sensiblement transversale.

Le dispositif 1 de transport comprend un organe 6 de préhension avec une tête 7 de préhension propre à être solidarisée avec la préforme 2, et préférentiellement, à être solidarisé de manière étanche avec son col 4.

Il est représenté sur la figure 1 un vêtissage dit « intérieur » de la préforme 2 et du type tel que décrit dans le document FR 2 794 109 précité, c'est-à-dire que la tête 7 de préhension est insérée à l'intérieur du col 4 et prend donc appui sur la face interne du col 4.

Toutefois, il convient de noter que le principe selon l'invention s'applique également à un vêtissage dit « extérieur » de la préforme 2, c'est-à-dire quand la tête 7 de préhension prend appui sur la face externe du col 4, en général en prenant appui sur le filetage 8 du col 4.

Selon l'invention, l'organe 6 de préhension présente au moins un alésage 9 débouchant à travers la tête 7 de préhension.

Ainsi, selon une première application de l'invention, la préforme 2 est apte à être désolidarisée de la tête 7 de préhension par injection d'un fluide sous-pression (préférentiellement de l'air comprimé sous une pression de l'ordre d'au moins plusieurs bars, de 2 à 5 bars) dans l'alésage 9 débouchant alors dans le volume 10 fermé qui est formé par le corps 3 de la préforme 2 solidarisée à ladite tête 7 et ce, que le volume 10 soit parfaitement étanche ou non.

De manière alternative, l'intérieur de la préforme 2 peut être aseptisé par injection d'un fluide aseptique dans l'alésage 9 débouchant dans le volume 10 fermé qui est formé par le corps 3 creux solidarisé à ladite tête 7.

De manière générale, il est possible d'injecter tout type de fluide à travers l'alésage 9 présentant une interaction appropriée et/ou une action de traitement souhaitée sur le volume 10 fermé qui est formé par le corps 3 creux solidarisé à la tête 7.

L'alésage 9 est donc prévu traversant l'organe 6 de préhension, de manière sensiblement rectiligne à l'intérieur dudit organe 6. Cet alésage 9 est de forme générale cylindrique et il est formé centralement dans l'organe 6, c'est-à-dire qu'en considérant que l'organe 6 de préhension définit un axe X-X principal de préhension, axe X-X par rapport auquel il est déterminé si la préforme 2 est correctement solidarisée à la tête 7 de préhension et alignée avec celle-ci, alors l'alésage 9 est sensiblement formé de manière coaxiale à l'axe X-X principal de préhension.

De manière plus précise, l'organe 6 de préhension comprend un mandrin 11 présentant ladite tête 7 de préhension ainsi qu'une tige 12 de préhension sur laquelle est fixé, préférentiellement de manière amovible, ledit mandrin 11.

L'alésage 9 est donc formé et creusé aussi bien dans la tige 12 de préhension que dans le mandrin 11.

Afin de limiter les pertes de charges lors de l'injection du fluide sous pression dans l'alésage, ledit alésage est prévu sensiblement rectiligne à l'intérieur de l'organe de préhension et il est, de manière avantageuse, de forme générale sensiblement cylindrique.

Plus précisément, l'alésage 9 présente une ouverture 13 amont pour l'injection d'un fluide ainsi qu'une ouverture 14 aval dans la tête 7 de préhension. L'ouverture 13 amont présente un rétrécissement de sa section transversale en direction de l'ouverture 14 aval.

Au niveau de l'ouverture 13 amont, afin de permettre à une quantité maximale de fluide d'être canalisée, dirigée et d'entrer dans l'alésage 9, il est donc prévu que l'ouverture 13 amont présente une section transversale plus grande que la section transversale de la majeure partie de l'alésage 9, et notamment plus grande que la section transversale de l'ouverture 14 aval.

Même s'il est représenté sur la figure 1 un dispositif 1 de transport avec un seul alésage 9 central, il est également possible de prévoir plusieurs alésages 9, par exemple disposés concentriquement à l'axe X-X principal de préhension, ou alors de prévoir un alésage 9 se divisant en plusieurs alésages secondaires tous débouchant dans la tête 7 de préhension.

La présente invention porte également, selon un second de ses aspects, et de manière plus générale, sur une installation de convoyage de corps 2 creux comprenant une pluralité de dispositifs 1 de transport qui circulent alignés les uns à la suite des autres sur une chaîne sans fin, non représentée, de type connu en soi, comme visible, par exemple, sur le document FR 2 872 805 précité.

L'installation de convoyage comprend des moyens d'injection d'un jet de fluide, tel qu'un fluide de soufflage sous-pression, par exemple à une pression comprise entre 2 et 5 bars, sachant que l'on trouve, sur ce type de machine, une pression qui est disponible jusqu'à 40 bars, lesquels moyens d'injection du fluide de soufflage sont dirigés vers un point de passage d'au moins un alésage 9 formé intérieurement dans un dispositif 1 de transport lors de sa circulation sur la chaîne sans fin.

De manière préférentielle, les moyens d'injection se présentent sous la forme d'une buse 15 de soufflage qui est fixe, solidaire du châssis de l'installation, non représenté, laquelle buse 15 est donc dirigée vers un point de passage de l'ouverture 13 amont de l'alésage 9 lors de la circulation des dispositifs 1 de transport associés à la chaîne sans fin.

Afin d'éviter une perte trop importante du fluide injecté par la buse 15, ladite buse 15 est située en regard d'un point de passage de l'ouverture 13 amont de l'alésage des dispositifs 1 de transport et elle n'est écartée que d'une très faible distance par rapport à cette ouverture 13 amont, par exemple de l'ordre du millimètre, voire quelques millimètres.

Selon une application de l'invention, l'installation de convoyage peut comprendre également des moyens de détection du genre caméra de reconnaissance de forme (non représentée sur la figure mais de type connu en soi), pour déceler un défaut d'alignement du corps 3 de la préforme 2 avec l'axe X-X principal de préhension et l'axe de la tête 7 de préhension, ce grâce à quoi il est déterminé si la préforme 2 transportée par ladite tête 7 de préhension doit être désolidarisée de cette dernière par l'injection du fluide de soufflage sous-pression.

La commande de cette injection peut être réalisée par le biais d'un automate qui reçoit et traite les informations provenant des moyens de détection dont il était question auparavant.

Cet automate comporte un programme aménagé pour organiser la commande de l'injection du fluide sous pression dans la préforme reconnue comme présentant un défaut susceptible de générer des incidents dans le four de chauffage de ces préformes.

L'aménagement du programme d'injection comporte une phase appropriée pour l'administration du fluide sous pression vers la préforme sélectionnée, laquelle phase s'étend, par exemple, sur une durée sensiblement supérieure au temps mis par ladite préforme sélectionnée pour passer devant la buse de projection de façon à garantir un temps d'injection dudit fluide sous pression dans l'alésage qui soit suffisamment long pour assurer l'éjection de ladite préforme sélectionnée.

Cette phase d'injection est également aménagée pour permettre une anticipation du déclenchement de l'injection du fluide sous pression dans ladite préforme indésirable face au passage de ladite préforme indésirable devant la buse 15 de projection dudit fluide et pour maintenir cette injection dudit fluide sous pression au moins jusqu'au passage complet de la tête 7 de préhension devant ladite buse 15 de projection dudit fluide.

L'invention permet donc, de manière générale, de réaliser un procédé de convoyage d'une préforme 2 solidarisée à un dispositif 1 de transport, procédé comprenant une étape d'injection d'un fluide à travers au moins un alésage 9 prévu traversant ledit dispositif 1 de transport et débouchant dans le volume 10 intérieur du corps 3 de la préforme 2 solidarisée audit dispositif 1 de transport.

Selon une autre forme d'application de l'invention, il est injecté un fluide aseptique à travers l'alésage 9. Alternativement, il est injecté un fluide sous-pression, ce qui permet de désolidariser le corps 2 creux de la tête 7 de vêtissage.

Ainsi, de manière résumée, selon une application de l'invention, il est détecté, à l'aide des moyens de détection, si une préforme 2 est désaxée de l'axe X-X ou présente un défaut de fabrication. Il est alors déterminé, à l'aide de l'automate et de programmes appropriés et/ou de moyens de calcul, quand le dispositif 1 de transport avec cette préforme, et plus précisément son alésage 9, va passer en regard des moyens 15 d'injection de fluide de soufflage, pour ensuite commander l'injection de ce fluide pour dévêtir la préforme (ou n'importe quel corps creux solidarisé sur une tête 7 de vêtissage).

Le temps de réponse des moyens d'injection de fluide est nettement plus rapide que les temps de réponse des moyens pneumatiques à piston selon l'art antérieur précité, ce qui permet de ne dévêtir qu'une seule préforme à la fois.

Ainsi, selon une première application de l'invention, le corps creux est apte à être désolidarisé de la tête de préhension par injection d'un fluide de soufflage sous-pression dans l'alésage débouchant alors dans le volume fermé qui est formé par le corps creux solidarisé à ladite tête.

De manière alternative, l'intérieur du corps creux peut être aseptisé par injection d'un fluide aseptique dans l'alésage débouchant dans le volume fermé qui est formé par le corps creux solidarisé à ladite tête.

De manière plus générale, l'invention peut trouver sa place sur une installation de transport de récipients à col, où il est parfois nécessaire de dévêtir les corps creux présentant un défaut, comme, par exemple, en sortie de l'unité de soufflage des préformes.

## Revendications

1. Procédé de convoyage d'un corps (2) creux solidarisé à un dispositif (1) de transport, **caractérisé en ce qu'**il comprend une étape d'injection d'un fluide aseptique à travers au moins un alésage (9) prévu traversant ledit dispositif (1) de transport et débouchant dans le volume (10) intérieur du corps (2) creux solidarisé audit dispositif (1) de transport.

2. Procédé selon la revendication 1, **caractérisé en ce que** le corps (2) creux est une préforme de récipient.

3. Dispositif (1) de transport d'un corps (2) creux, du type préforme en matière thermoplastique, comprenant un organe (6) de préhension avec une tête (7) de préhension propre à être solidarisée avec ledit corps (2) creux, dispositif **caractérisé en ce que** ledit organe (6) de préhension présente au moins un alésage (9) débouchant à travers ladite tête (7) de préhension dans le volume (10) fermé qui est formé par le corps (3) creux solidarisé à ladite tête (7) et **en ce qu'**il comporte des moyens d'injection d'un fluide aseptique à l'intérieur du corps (2) creux au travers de l'alésage (9).

4. Dispositif selon la revendication 3, **caractérisé en ce que** l'alésage (9) présente une ouverture (13) amont pour l'injection du fluide ainsi qu'une ouverture (14) aval dans ladite tête (7) de préhension, l'ouverture amont (13) présentant un rétrécissement de sa section transversale en direction de l'ouverture (14) aval.

5. Dispositif selon la revendication 3, **caractérisé en ce que** l'alésage (9) est prévu 3 sensiblement rectiligne à l'intérieur dudit organe (6) de préhension.

6. Dispositif selon l'une quelconque des revendications 3 ou 4, **caractérisé en ce que** l'alésage (9) est de forme générale sensiblement cylindrique.

7. Installation de convoyage de corps creux **caractérisée en ce qu'**elle comprend une pluralité de dispositifs (1) de transport selon l'une quelconque des revendications 3 à 6 circulant alignés les uns à la suite des autres sur une chaîne sans fin, chaque dispositif (1) de transport comprenant un organe (6) de préhension d'un corps (2) creux présentant au moins un alésage (9) débouchant à travers une tête (7) de préhension dans le volume (10) fermé qui est formé par le corps (3) creux solidarisé à ladite tête (7) et **en ce qu'**il comporte des moyens (15) d'injection d'un fluide aseptique à l'intérieur du corps (2) creux au travers de l'alésage (9).

8. Installation selon la revendication 7, **caractérisée en ce que** lesdits moyens (15) d'injection se présentent sous la forme d'une buse de soufflage.

## Claims

1. Method for conveying a hollow body (2)secured to a transport device (1), **characterized in that** it comprises a step of injection of a disinfecting fluid through at least one bore (9) designed to pass through said transport device (1) and opening into the inner volume (10) of the hollow body (2) secured to said transport device (1).

2. Method according to claim 1, **characterized in that** the hollow body (2) is a preform of a container.

3. Device (1) for transporting a hollow body (2), such as a preform made of thermoplastic material, comprising a gripping member (6) with a gripping head (7) able to be secured to the hollow body (2), the device being **characterized in that** said gripping member (6) has at least one bore (9) opening through the gripping head (7) into the closed volume (10) which is formed by the hollow body (2) secured to the head (7) and **in that** it comprises means for injecting a disinfecting fluid into the hollow body (2) through the bore (9).

4. Device according to claim 3, **characterized in that** the bore (9) has an upstream opening (13) for the injection of the fluid and a downstream opening (14) in the gripping head (7), the upstream opening (13) having a narrowing of its transversal section in the direction of the downstream opening (14).

5. Device according to claim 3, **characterized in that** the bore (9) is provided substantially straight into said gripping member (6).

6. Device according to any one of the claims 3 or 4, **characterized in that** the bore (9) has a substantially cylindrical general shape.

7. Installation for conveying a hollow body **characterized in that** it comprises a plurality of transporting devices (1) according to any one of claims 3 to 6 moving aligned one after the other on an endless belt, each transporting device (1) comprising a gripping member (6) of a hollow body (2) having at least one bore (9) opening trough a gripping head (7) into the closed volume which is formed by the hollow body (2) secured to said gripping head (7) and **in that** it comprises injection means (15) of a disinfecting fluid into the hollow body (2) through the bore (9).

8. Installation according to claim 7, **characterized in that** said injection means (15) has the shape of a blowing nozzle.

## Patentansprüche

1. Verfahren zur Beförderung eines Hohlkörpers (2), der mit einer Transporteinrichtung (1) verbunden ist, **dadurch gekennzeichnet, dass** es einen Schritt des Einspritzens eines aseptischen Fluids durch mindestens eine Bohrung (9) umfasst, die durch die Transporteinrichtung (1) hindurchgeht und in dem Innenvolumen (10) des Hohlkörpers (2) mündet, der mit der Transporteinrichtung (1) verbunden ist.

2. Verfahren nach Anspruch 1, **dadurch gekennzeichnet, dass** der Hohlkörper (2) eine Behältervorform ist.

3. Fördereinrichtung (1) für einen Hohlkörper (2) des Typs Vorform aus Thermoplast, umfassend ein Greifelement (6) mit einem Greifkopf (7), der mit dem Hohlkörper (2) verbunden werden kann, wobei die Einrichtung **dadurch gekennzeichnet ist, dass** das Greifelement (6) mindestens eine Bohrung (9) aufweist, die durch den Greifkopf (7) hindurch in dem geschlossenen Volumen (10) mündet, das von dem mit dem Kopf (7) verbundenen Hohlkörper (3) gebildet ist, und dass es Mittel zum Einspritzen eines aseptischen Fluids in den Hohlkörper (2) durch die Bohrung (9) umfasst.

4. Einrichtung nach Anspruch 3, **dadurch gekennzeichnet, dass** die Bohrung (9) eine stromaufwärtige Öffnung (13) zum Einspritzen eines Fluids sowie eine stromabwärtige Öffnung (14) im Greifkopf (7) aufweist, wobei die stromaufwärtige Öffnung (13) eine Verengung ihres Querschnitts in Richtung der stromabwärtigen Öffnung (14) aufweist.

5. Einrichtung nach Anspruch 3, **dadurch gekennzeichnet, dass** die Bohrung (9) im Wesentlichen gerade im Inneren des Greifelements (6) vorgesehen ist.

6. Einrichtung nach einem der Ansprüche 3 oder 4, **dadurch gekennzeichnet, dass** die Bohrung (9) eine im Wesentlichen zylindrische allgemeine Form aufweist.

7. Anlage zur Beförderung von Hohlkörpern, **dadurch gekennzeichnet, dass** sie eine Vielzahl von Fördereinrichtungen (1) nach einem der Ansprüche 3 bis 6 umfasst, die nacheinander auf einer Endloskette umlaufen, wobei jede Fördereinrichtung (1) ein Greifelement (6) für einen Hohlkörper (2) umfasst, das mindestens eine Bohrung (9) aufweist, die durch einen Greifkopf (7) in dem geschlossenen Volumen (10) mündet, das von dem mit dem Kopf (7) verbundenen Hohlkörper (3) gebildet ist, und dass sie Mittel (15) zum Einspritzen eines aseptischen Fluids in das Innere des Hohlkörpers (2) durch die Bohrung (9) umfasst.

8. Anlage nach Anspruch 7, **dadurch gekennzeichnet, dass** die Einspritzmittel (15) in Form einer Einblasdüse vorhanden sind.
